Europäisches Patentamt

European Patent Office

Office européen des brevets

(19)

(11) Publication number: **0 378 507**
**A1**

## EUROPEAN PATENT APPLICATION

(21) Application number: 90630017.3

(22) Date of filing: 12.01.90

(51) Int. Cl.5: **A23K 1/10, A23N 17/00,**
**C12M 1/36, A01C 3/02**

(30) Priority: **12.01.89 IL 88937**

(43) Date of publication of application:
**18.07.90 Bulletin 90/29**

(84) Designated Contracting States:
**AT BE CH DE DK ES FR GB GR IT LI LU NL SE**

(71) Applicant: **Zisser, Mordechai**
**Beit Maya 75 Petach Tikvah Road**
**Tel Aviv(IL)**

Applicant: **CANDAR INVESTMENTS INC.**
**Bank America Building 50th Street**
**Panama(PA)**

(72) Inventor: **Zisser, Mordechai**
**75 Petach Tikvah Road**
**Tel Aviv(IL)**

(74) Representative: **Waxweiler, Jean et al**
**OFFICE DENNEMEYER S.à.r.l. P.O. Box 1502**
**L-1015 Luxembourg(LU)**

(54) **A method of manufacturing feed for livestock and poultry.**

(57) A process for the manufacture of feed for livestock and poultry which comprises: subjecting moist organic waste material to aerobic fermentation conditions at an operating temperature within the range of 70 to 85 C, until it is substantially free from pathogenic microorganisms.

FIG.1

FIELD OF THE INVENTION

The present invention relates to a process and system for the manufacture of feed for livestock and poultry.

BACKGROUND OF THE INVENTION

The economic use of resources is widely recognized as one of the needs of the present day. One way in which such economy can be practiced is in the recycling of waste products. Animal and poultry feeds consist to some extent of products manufactured from primary crops grown specially for the purpose, although waste materials are also used to a limited extent. One drawback of presently used waste materials is that they consume a great deal of energy in drying them. Agricultural waste, presently used mainly for fertilizers, could be economically recycled for use in manufacturing feed, were it not for the fact that such waste often contains infective agents which create health risks and for this reason, in the past, infection from such recycled waste has had catastrophic effects.

SUMMARY OF THE INVENTION

It is an object of the present invention to utilize organic waste products for the manufacture of feeds for livestock and poultry.

Another object of the invention lies in the provision of a manufacturing process which will achieve such an object.

Yet another object of the invention is to provide such a process which will not put the health at risk, of livestock and poultry which consume such feeds.

Still another object of the invention is to provide such a process which is economical and which in particular is not energy intensive.

A further object of the invention is to provide highly efficient apparatus for putting into practice the process of the invention.

Other objects of the invention will appear from the description which follows.

The present invention accordingly provides in one embodiment a process for the manufacture of feed for livestock and poultry comprises subjecting moist organic waste material to aerobic fermentation conditions at an operating temperature within the range of 70 to 85°C, until it is substantially free from pathogenic microorganisms.

The term "substantially free from pathogenic microorganisms" means that pathogenic levels are reduced to a level at which they will not damage livestock when the product feed is fed thereto, and especially when reduced to below the enumeration level, as understood by persons skilled in the art.

It has been found that within the recited temperature range a period of about one to two days generally suffices to make the waste material substantially free of pathogenic organisms. Therefore, according to another embodiment of the invention, a process for the manufacture of feed for livestock and poultry comprises subjecting moist organic waste material to aerobic fermentation conditions for one to two days at an operating temperature within the range of 70 to 85°C.

BRIEF DESCRIPTION OF THE DRAWINGS

The present invention will be more fully understood and appreciated from the following detailed description, taken in conjunction with the drawings, in which:

Fig. 1 is a schematic plan view of the floor of a fermentation unit, constructed in accordance with an embodiment of the present invention;

Fig. 2 is a schematic cross-sectional illustration of the fermentation unit partially depicted in Fig. 1, taken in the direction of line II-II therein;

Fig. 3 is an enlarged view of detail III shown in Fig. 1;

Fig. 4 is a schematic, partially cut-away, end elevational view of the fermentation unit partially depicted in Fig. 1;

Fig. 5 is a perspective view of a portion of an air supply pipe depicted in Figs. 3 and 4; and

Fig. 6 is a block diagram illustration of a computer-controlled system for the manufacture of feed for livestock and poultry, in accordance with an embodiment of the present invention.

## DETAILED DESCRIPTION OF THE INVENTION

The present invention provides in one embodiment a process for the manufacture of feed for livestock and poultry from a moist organic material. The initial moist organic material may comprise e.g. between 50 and 90 wt.% dry material; the initial material may comprise faeces, which preferably constitutes at least 50% thereof, based on the weight of the dry material. Also, lime may be present in an amount between 0.1 and 1.0 (e.g. about 0.5) wt.% of the dry material. In a preferred embodiment of the invention, the operating temperature may be maintained, and/or the moist organic waste material may be raised from ambient temperature to the operating temperature by controlling access of air to the moist organic waste material.

By the term "faeces" in the present context, there is intended principally either (e.g.) poultry litter, or excrement from cattle. In accordance with a presently preferred embodiment of the invention, the initial material comprises 55 to 99.9wt.% poultry litter, 0 to 44.9wt.% other organic waste material and preferably 0.1 to 1.0 wt.% lime, based on the weight of dry material. The other organic waste material may include e.g. food waste, vegetable and food waste, byproducts of the food industry, kitchen waste, and fermentable abattoir waste particularly rumen content. A small proportion of waste from citrus processing e.g. 0.5 - 5.0 wt.% based on the dry weight of the total composition, has been found to be advantageous.

Although animal or poultry excrement is a presently preferred ingredient of the initial material, the present invention can utilize many other organic waste materials. By way of example only, initial materials including wood chips, sawdust, paper mill waste, bark, sludge, fruit pulp, fermentable abattoir waste and kitchen waste, have been successfully converted. Spoiled grains, fruit and vegetable wastes, other crop wastes, poultry offal, spoiled meat, bakery wastes, beer process wastes, coffee wastes, dairy wastes, and food processing wastes, for example, may all be included in the initial material to be subjected to the process of the present invention.

As stated, the process of the invention is effected under aerobic conditions. It is believed that fermentation takes place under the influence of thermophilic microorganisms present in the initial material. Such conditions may for example include supplying air in intermittent pulses to the moist organic waste material, the temperature of which is raised from ambient temperature to the operating temperature by control of the intermittent air supply, and the operating temperature is also maintained by control of the intermittent air supply. Apparatus for supplying air is described below in conjunction with the drawings.

It is also presently preferred that in such an embodiment, in accordance with one alternative, the process includes the steps of monitoring the temperature at a plurality of points in the moist organic waste material at predetermined time intervals, and adjusting in a predetermined manner the intermittent air supply as to the intervals between pulses and as to the length of each pulse, in order to maintain differences in the temperature monitored at successive time intervals within predetermined limits.

In another alternative, the process includes the steps of monitoring both the oxygen content of the ambient gases, and the temperature, at a plurality of points in the moist organic waste material at predetermined time intervals, and adjusting in a predetermined manner the intermittent air supply as to the intervals between pulses and as to the length of each pulse, when the oxygen content of the preceding pulse has been depleted to a predetermined extent, in order to maintain differences in the temperature monitored at successive time intervals within predetermined limits.

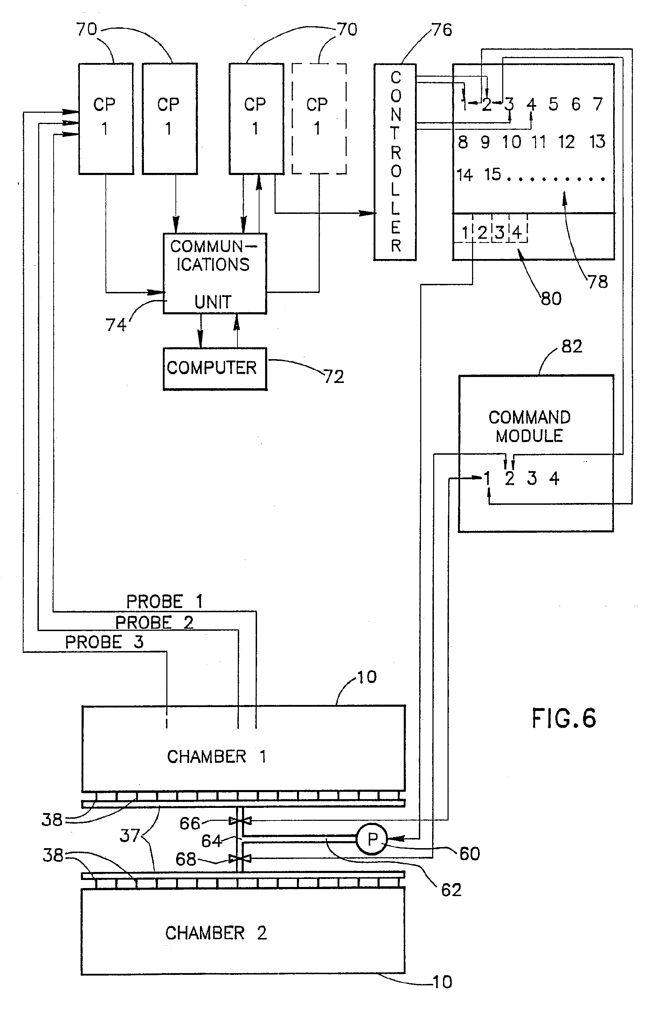

The process of the invention may conveniently employ programmed computerized control apparatus, such as described below in conjunction with Fig. 6, in such manner that the step of adjusting in a predetermined manner comprises control by output from this apparatus, while the input thereto comprises data obtained from the monitoring step.

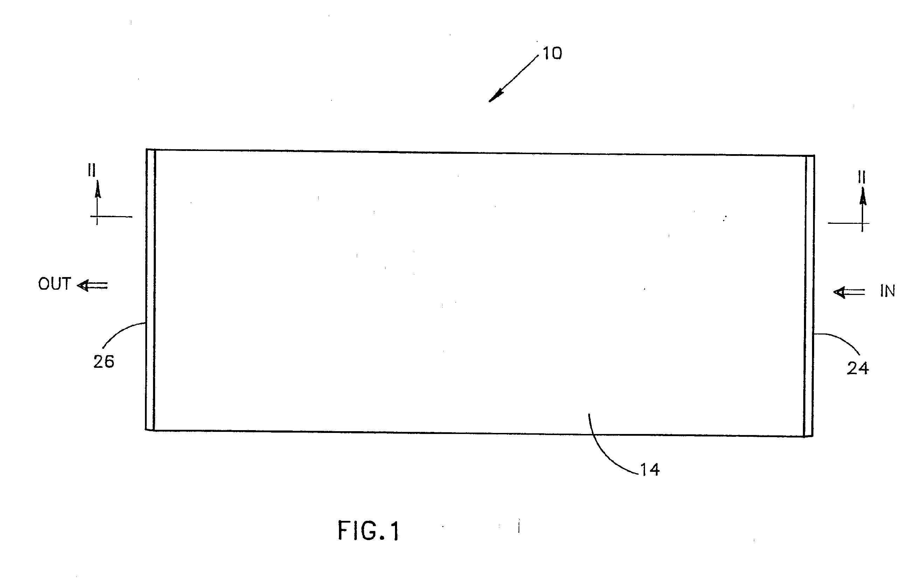

Reference is now made to the drawings, in which there is shown a system for implementing the manufacturing process of the present invention. With particular reference to Figs. 1-4, a preferred fermentation unit in which the process of the present invention may be carried out is fermentation chamber 10. Chamber 10 is typically rectangular in shape and has a holding capacity of approximately 35 tonnes

3

and is constructed so as to facilitate complete control of the fermentation process. Therefore, not only is air supplied under control so as to enable temperature control within the chamber, but it is also necessary that every wall of the fermentation chamber be insulated.

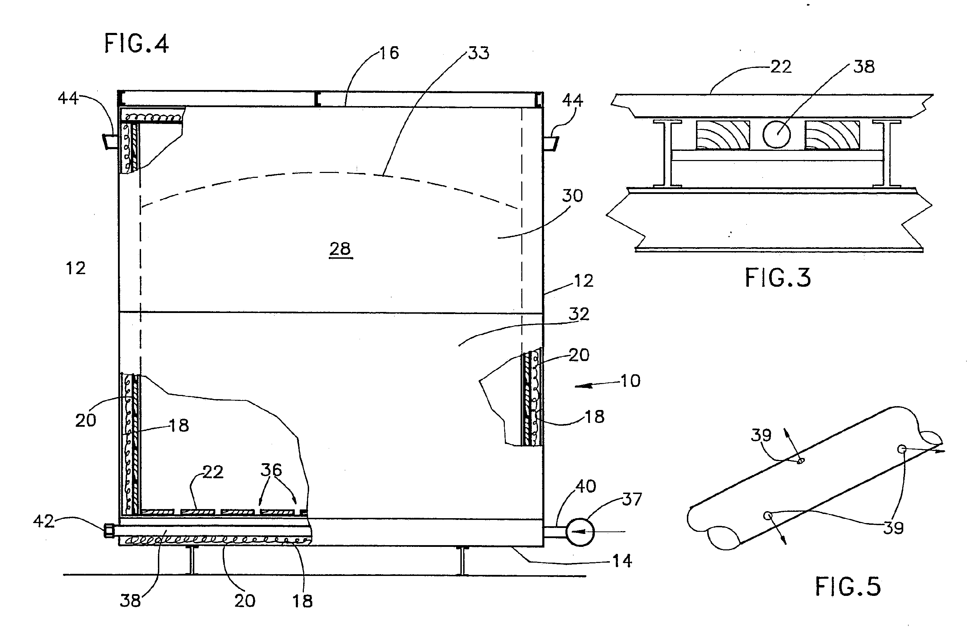

To this end and with particular reference to Fig. 4, side walls 12, floor 14 and roof 16 of chamber 10 are insulated and are typically formed of structural sheet metal coated on an inward-facing surface 18 with a layer 20 of polyurethane and lined with wooden boards 22. A preferred maximum U-value of the wall structure of the chamber 10 is approximately 0.2794 kcal/m$^2$°C/h.

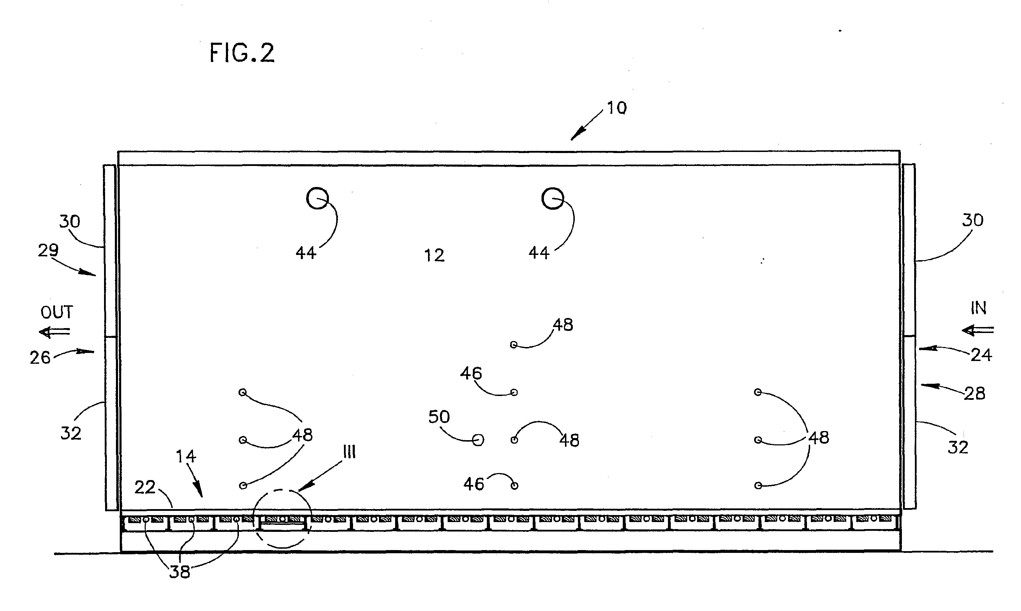

Chamber 10 defines predesignated entry and exit ports, shown, for example, at respective ends 24 and 26 (Figs. 1 and 2). It will be appreciated that complete functional separation of the two ends of the chamber must be ensured so as to prevent infection of processed material by non-processed material.

An entry door 28 and an exit door 29, provided at respective entry and exit ports 24 and 26, are divided into upper and lower sections, respectively referenced 30 and 32. The use of a divided door facilitates greater filling of the chamber as lower section 32 is closed after the level of material loaded into chamber 10 reaches a predetermined height, so as to permit material to be piled up against the lower section 32 while material is still being loaded through the upper half of the door. Loading of the material into chamber 10 may be achieved by any suitable loading apparatus, such as a tractor.

Once the maximum height of material within the fermentation chamber has been reached, as shown by broken line 33 in Fig. 4, upper section 30 of the door is also closed.

With particular reference now to Figs. 2, 3 and 4, the floor 14 of chamber 10 comprises a layer of the wooden boards 22 arranged typically lengthways and so as to define gaps 36 (Fig. 4) between them.

A plurality of air conduits 38 is arranged transversely to the wooden boards and therebeneath for the supply of air, as described above, to the material being processed. As shown in Fig. 5, each conduit 38 comprises a number of air supply holes 39 spaced along its length and, as shown in Fig. 4, in registration with the gaps 36 between the wooden boards. Alternate holes are inclined relative to each other at a predetermined angle relative to a vertical direction, as shown, in order to reduce the likelihood of blockage by the material being processed.

Air is supplied via a relatively large diameter-pipe 37 to an inlet 40 (Fig. 4) of each conduit 38. Distal ends of conduits 38 are closed by removable caps 42. In a preferred embodiment of the invention, air may be supplied at a rate of at up to 1500-1800 m$^3$/h and at a pressure, typically, of up to 0.5-0.6 atm., although maybe as high at 2.0 atm..

There are further provided, in each side wall 12, a number of air outlet pipes 44. These are typically large diameter pipes that permit free exchange of air between the inside of fermentation chamber 10 and the outside, so as to ensure that the pressure within the chamber is maintained at substantially atmospheric pressure. It has been found that the effect on the fermentation process of the air exchange between the inside of the chamber and the outside, that occurs via pipes 44, is negligible.

As mentioned above, the fermentation process is very closely monitored, preferably by means of processing apparatus, such as an IBM-compatible personal computer. As stated above, by controlling the air supply to the interior of fermentation chamber 10, the temperature of the material is controlled and therefore, the entire process is controlled. It is, therefore, necessary to monitor the temperature of the material.

Temperature monitoring is facilitated by the provision of typically three thermocouples associated with the computer. The thermocouples are preferably incorporated into elongate probes which are inserted into the chamber via holes formed in side walls 12 thereof. It has been found that a pair of fixed-position thermocouples 46, typically located approximately halfway along the chamber length and a third thermocouple, placed at any one of the positions referenced 48, provide accurate data with regard to the temperature of the material.

In order to check the progress of the fermentation process, it is necessary to sample the material contained by fermentation chamber 10. There is provided, therefore, an inspection port 50 through which samples of material may be removed for analysis of, inter alia, protein, pH and ash content.

As already mentioned, a highly efficient way of carrying out the process of the invention is by computer control. Reference is thus made to Fig. 6, which is a block diagram illustration of a computer-controlled system for the manufacture of feed for livestock and poultry, in accordance with an embodiment of the present invention.

As shown, a pair of similar chambers 10 typically receive their air supply from a common pump 60 which may be any suitable typically 2.25 horse power pump, having a pumping capacity of up to 1800 m$^3$/h. A first air supply conduit 62 is connected to main supply pipes 37 via a second conduit 64 along which are provided separately operable first and second valves, respectively referenced 66 and 68 and respectively associated with chamber 1 and chamber 2. Valves 66 and 68 are preferably hydraulically operated valves.

There are two main factors that determine the process of the present invention, namely, keeping the temperature of the material inside the fermentation chambers within a desired range and keeping the oxygen content of the material being processed within a desired range.

According to a preferred embodiment of the invention, as there exists, in general terms, a correlation between the temperature and the oxygen content of the material, only the temperature of the material is monitored and controlled on a continous basis, the oxygen content of the material being predicted according to tables compiled by the applicants based on tests carried out by them. Although the oxygen content of the material is periodically sampled, this is not on a regular basis and merely serves to confirm that the measured oxygen content is approximately equal to that predicted.

Thus signal outputs (Probes "1" and "2") from fixed position thermocouples 46 and a third output (Probe "3") from the third thermocouple are provided as inputs to a predetermined control panel, referenced 70. A typical control panel suitable for use in the invention is a Picco-Logo 100 control panel made by Lombrozo Electronic Engineering Ltd., of 7, Arvei Nahal Street, Ramat Gan, Israel.

Each of control panels 70 provides an output to a central computer 72 via a communications unit 74. Although only a pair of chambers 10 is shown, three or more pairs of chambers may be employed and controlled by a single central computer 72, which typically is an 640 K IBM-compatible personal computer, as stated. A typical communications unit 74 suitable for use in the invention is as made by Lombrozo Electronic Engineering Ltd, Israel.

A preferred mode of operations of computer 72 is provided in the form of a program listing in Appendix I.

According to 'Routine I' of the computer listing, at predetermined intervals, such as every 30 seconds, computer 72 receives temperature readings from the thermocouples, via control panel 70 and communications unit 74. In a system employing a number of chambers so as to necessitate use of more than one control panel, only one of the control panels, (in the shown example, control panel "3") receives an output from the computer. The output comprises signals operating the pump and hydraulic valves.

As the computer receives information related to the temperature measured in each fermentation chamber, the temperature change over a period of time is determined. If after a predetermined period of time, for example, twenty minutes, a negative temperature trend is detected in, for example, chamber 1, for that chamber, the computer switches to 'Routine II' of the program (Appendix I), which is described in detail hereinbelow. As shown in the computer listing, once any necessary adjustment to the control of the chamber has been made, that chamber is automatically switched back to control via Routine I.

A possible decision of the computer in seeking to overcome a negative temperature trend is to pump air into the chamber. A command to operate pump 60 and open valve 66 is transmitted as an output signal to a controller 76, such as a Picco Controller, manufactured by Lombrozo Electronic Engineering Ltd, Israel, via communications unit 74 and control panel 3. A plurality of relay switches, shown generally at 78, are associated with outputs from controller 76, as are a plurality of connectors 80. Typical relay switches that may be used are 8 pin, 220 V AC, 10 Amp, 0.25 hp, 50-60 Hz, 220 VAG relay switches as made by Rele Ltd, Italy, on a 580 V, type S8 50 base, also made by Rele Ltd. Any other suitable relay equipment may alternatively be used. A typical connector is model LCT DO 99 A65, made by Contactor Telemecanique, France.

Individual relay switches, referenced 1,2,3....etc are associated, via a command module 82, with predetermined hydraulic valves. Command module 82 comprises an arrangement of solenoid valves shown as 1,2,3....etc, each of which is operable by a similarly referenced relay switch at 78.

Therefore, valve 66 is opened (and closed when appropriate) by means of solenoid 1 as it receives a signal from relay switch 1. At the same time, a signal via connector 1 (in the present example) is provided to actuate pump 60. Pump 60 is also operable by means of connector 2, as would occur in a situation when it is sought to supply air only to chamber 2.

In addition to the continuous checking of the temperature trends in each of the chambers, as described above, a further, routine assessment of the overall operation of each of the chambers is also made at predetermined intervals. This is shown as 'Routine II' in the computer listing (Appendix I). Each of the fermentation chambers constitutes part of a 'set', each set being controlled by the computer independently of the other sets. According to the present embodiment, a single set includes up to six chambers arranged in three pairs of two, there typically being up to six sets of chambers controlled by a single computer. In each complete set of six chambers, for example, according to Routine II, the temperature trend of each chamber is checked once every ninety minutes.

During this period, the temperature trend is determined for the chamber concerned and, if the rate of the rise in temperature is found to be lower than desired, even though it may be positive, air may be supplied to the chamber. The rate at which the air is supplied and the time over which it is supplied will be determined by the computer according to the actual temperature of the material being processed, the rate of rise of the temperature and the rate it is sought to achieve. Preferably, the fermentation process is terminated by the computer about 48 hours after a temperature of 70 degrees celsius has been reached.

In accordance with an alternative embodiment of the invention, an oxygen sampling device (not shown) in the form of a probe is provided at one of locations 48 adjacent holes 46 in the side wall 12 of each chamber 10 (Fig. 2). A typical sampling device suitable for use in the present invention is an AMF Oxygen Indicator Model 468838 manufactured by BIOTEC-JAMAICA Inc., of Franklin, Nebraska, U.S.A., equipped with an AMF Oxygen Sensor Cell Model 45761 1, also made by Biotec-Jamaica.

According to a preferred method of operation, the computer receives a reading of oxygen in each of the chambers every thirty seconds. As soon as the oxygen level drops below 6% an any given chamber, the pump associated with that chamber is operated so as to raise the oxygen level to 18%.

Temperature levels in the chambers are also measured, as described above. In the routine temperature check that is carried out every 90 minutes, if the rate of increase in the temperature in a given chamber is found to be less than 0.6 degree celsius per hour, then the computer transfers to a program which monitors the chamber temperature only. Once the rate of temperature increase rises above 0.8 degrees per hour, the oxygen content in the chamber is once again monitored.

If, when the temperature in the chamber is lower than 68 degrees celsius and subsequent to the return to oxygen monitoring, the rate of increase in temperature again falls below 0.6 degrees per hour, the computer transfers to operate the 'temperature only' program. In any event, once the temperature in a chamber rises above 68 degrees, only the temperature is monitored.

The present invention will be illustrated by the following non-limiting Examples.


## EXAMPLES


### General procedure

Organic waste material was sorted manually and mechanically (e.g. magnetically) in order to remove foreign bodies. It was thoroughly mixed in a reserved site of the demonstration plant and introduced into fermentation units (pilot scale units of capacity 300 kg. or full scale units of 35 tons capacity), under sufficient pressure to compress it to an extent which would nevertheless allow air free access. The floor of the fermentation units consisted of a wooden grid covered by a layer of sawdust. Air could be injected through the floor via a pipe system which terminated in a plurality of injection vents disposed in the spaces between the wooden planks of the grid.

It is desirable to age the mixture (e.g. up to about 3 hours) either prior to introduction to the fermentation units, or after introduction thereto but prior to the injection of oxygen.


### Materials

Poultry litter was used alone or in combination with either rumen material or kitchen waste. The poultry litter consisted of broiler litter on wood shavings or shredded paper waste basis collected from commercial broiler farms after one cycle of fattening. Rumen material was collected from fresh opened rumen in the intestine room of commercial abattoirs. Kitchen waste was collected from large army kitchens.


### Process conditions

The material in the fermentation units was heated slowly by injection of air. In a first stage the material was allowed to rise from ambient temperature to a minimum of 70°C over a period of about 8-48 hours. The temperature was monitored at various points in the mass of material and the rate of heating was controlled by pulsing in air at intervals. In general, the intervals were such that the oxygen content of the air from one pulse in monitored locations in the mass of material had been depleted from about 21 to about 6%, before adding air in a succeeding pulse. It is generally preferred that such intervals are no longer than ten minutes. The length of the pulse could be either relatively shortened or lengthened so as to respectively slow or accelerate the rate of heating to reach the required minimum temperature in the stated period of time, in all monitored locations. Thereafter the heating was similarly controlled to maintain the temperature within a range of 70 to 85° for a period of about 32 to 48 hours, the total time of passage of air in general terms being 40-96 hours.

EXAMPLE I: Fermentation of Poultry Litter.

Poultry litter was subjected to the process of the invention according to the details set out above. Table A shows the count of microorganisms before and after processing. It is seen that the present process affects mainly Coliform bacteria, Clostridia, yeasts and molds, since their counts were reduced to below the enumeration level and no Salmonella bacteria could be detected in the product. Mesophilic bacteria counts were generally reduced by 1-4 logarithmic units while thermoduric bacteria and total spores were not affected significantly. In addition to the tabulated results, it has been determined that effectively the same results are obtained by analyzing products taken from different locations in a particular fermentation unit.

The following is a typical analysis of a product of the present process, from poultry litter:

| Protein | 29% |
|---|---|
| Fat | 3% |
| Ash | 19% |
| Calcium | 3.5% |
| Phosphorus | 2.5% |
| Crude fiber | 16% |
| pH | 7.5 |
| Mcal ME / kg. | 2.0 |

In Tables A-C, the bacterial count figures in the product, obtained by subjecting the feed to the process of the present invention, meet the standards set by the Veterinary Service of the Government of the State of Israel.

TABLE A

| Microorganism count in treated poultry litter | | | | | | | | |
|---|---|---|---|---|---|---|---|---|
| RUN | Bacterial count (decimal logarithm) | | | | | | | |
| | total mesoph | total thermo | total spores | Enterococci* | Coliforms* | Clostridia? | yeast, molds* | Salmonella¡ |
| 1 b | 8.2 | 4.9 | 4.9 | 3.0 | <1 | 1.9 | <1 | - |
| a | 6.6 | 4.7 | 5.5 | 3.0 | <1 | 1.0 | <1 | - |
| 2 b | 8.5 | 5.2 | 5.1 | 3.2 | 3.2 | 2.2 | 3.3 | - |
| a | 5.0 | 4.0 | 3.3 | 3.0 | <1 | <1 | <1 | - |
| 3 b | 8.1 | 5.7 | 5.2 | 5.4 | 1.6 | 1.0 | 2.7 | - |
| a | 6.2 | 4.4 | 3.7 | 3.0 | <1 | <1 | <1 | - |
| 4 b | 8.9 | 5.4 | 5.3 | 5.2 | 1.0 | <1 | 1.5 | - |
| a | 7.3 | 4.3 | 5.1 | 3.0 | <1 | <1 | <1 | - |
| 5 b | 8.6 | 5.8 | 5.7 | 5.7 | 3.4 | 2.0 | 3.6 | - |
| a | 6.5 | 4.0 | 4.7 | 3.0 | <1 | <1 | <1 | - |
| 6 b | 6.8 | 5.7 | 5.1 | 3.5 | 1.7 | 2.0 | 2.7 | - |
| a | 5.0 | 4.7 | 4.5 | 3.0 | <1 | <1 | <1 | - |
| 7 b | 7.3 | 8.8 | 5.3 | 3.0 | <1 | <1 | <1 | - |
| a | 6.3 | 5.5 | 5.1 | <1 | <1 | <1 | <1 | - |
| 8 b | 9.3 | 5.5 | 5.6 | 6.7 | <1 | 1.7 | 3.1 | - |
| a | 5.0 | 3.6 | 3.0 | <1 | <1 | <1 | <1 | - |
| 9 b | 9.5 | 5.7 | 6.3 | 5.9 | <1 | 2.4 | 3.5 | - |
| a | 5.0 | 4.7 | 4.8 | <1 | <1 | <1 | <1 | - |
| 10 b | 8.3 | 5.2 | 5.2 | 4.7 | <1 | <1 | <1 | + |
| a | 5.0 | 5.9 | 3.9 | <1 | <1 | <1 | <1 | - |
| 11 b | 7.5 | 5.2 | 5.4 | 4.6 | <1 | <1 | 2.7 | - |
| a | 5.0 | 4.3 | 4.1 | <1 | <1 | <1 | <1 | - |
| 12 b | 8.9 | 5.5 | 5.3 | <1 | <1 | <1 | <1 | - |
| a | 6.6 | 5.5 | 5.2 | <1 | <1 | <1 | <1 | - |
| 13 b | 7.8 | 4.6 | 5.0 | 4.4 | <1 | <1 | <1 | - |
| a | 6.0 | 5.8 | 5.1 | <1 | <1 | <1 | <1 | - |
| KEY TO TABLES A, B and C: | | | | | | | | |
| mesoph = aerobic mesophilic (37oC) bacterial count<br>thermo = aerobic thermoduric (55oC) bacterial count<br>spores = bacterial count (37oC) after treatment at 80oC (10 mins) | | | | | | | | |

*total count
¡presence of Salmonella in 20 g. material
?total count of sulfite-reducing Clostridia, mainly Cl. welchii
b = before a = after aerob = aerobic bacteria

EXAMPLE II: Fermentation of Poultry Litter/Kitchen Waste.

A mixture of poultry litter (80%) with kitchen waste (20%) from two different sources was subjected to the present process according to the details set out above. For this purpose there were utilized 10 fermentation units each containing 35 tons of material. The results are given in table B. It may seen from the Table that the counts of Enterococci, Coliform bacteria, Clostridia, and yeasts and molds were reduced to below the enumeration level and no Salmonella bacteria could be detected in the product. Mesophilic bacteria counts were generally reduced by 3-4 logarithmic units (99.99%) while thermoduric bacteria and total spores were reduced slightly by 1-2 logarithmic units.

### TABLE B

| Microorganism count in poultry litter/kitchen waste | | | | | | | | |
|---|---|---|---|---|---|---|---|---|
| RUN | Bacterial count (decimal logarithm) | | | | | | | |
| | total mesoph | total thermo | total spores | Enterococci* | Coliforms* | Clostridia? | yeast, molds* | Salmonella/ |
| a | 8.2 | 5.0 | 5.3 | <1 | 3.7 | 2.4 | 4.5 | - |
| B | 7.8 | 6.6 | 5.6 | 3.8 | <1 | 1.9 | 3.3 | - |
| 1 b | 8.1 | 4.3 | 5.0 | 2.3 | <1 | 1.7 | <1 | - |
| a | 5.0 | 3.3 | 3.3 | <1 | <1 | <1 | <1 | - |
| a | 5.0 | 4.9 | 4.7 | <1 | <1 | <1 | <1 | - |
| a | 5.0 | 3.3 | 3.0 | <1 | <1 | <1 | <1 | - |
| 2 b | 8.7 | 4.3 | 5.5 | 5.7 | <1 | 1.3 | <1 | - |
| a | 4.0 | 3.1 | 3.3 | <1 | <1 | <1 | <1 | - |
| a | 4.0 | 3.1 | 3.3 | <1 | <1 | <1 | <1 | - |
| a | 5.0 | 3.9 | 4.9 | <1 | <1 | <1 | <1 | - |
| a | 5.0 | 4.3 | 4.3 | <1 | <1 | <1 | <1 | - |
| 3 b | 8.5 | 5.8 | 5.0 | 5.3 | 1.3 | 2.3 | 3.7 | - |
| a | 4.0 | 3.9 | 4.0 | <1 | <1 | <1 | <1 | - |
| a | 4.0 | 3.3 | 3.5 | <1 | <1 | <1 | <1 | - |
| a | 4.7 | 4.3 | 4.2 | <1 | <1 | <1 | <1 | - |
| a | 4.0 | 3.0 | 3.5 | <1 | <1 | <1 | <1 | - |
| a,B = kitchen waste only from two different sources<br>Note: in each run on the mixture, samples were collected from 3 or 4 different locations in the fermentation units. | | | | | | | | |

EXAMPLE III: Fermentation of Poultry Litter/Rumen Content.

A mixture of poultry litter (80%) with rumen content (20%) from three different abattoir sources was subjected to the present process according to the details set out above, using pilot plant scale fermentors. The results are given in table C. It may seen from the Table that the counts of Coliform bacteria, Clostridia, and yeasts and molds were reduced to below the enumeration level and no Salmonella bacteria could be detected in the product. Total aerobic bacteria counts were generally reduced by 3-5 logarithmic units while thermoduric bacteria and total spores were reduced by 2-4 logarithmic units.

TABLE C

| Microorganism count in poultry litter/rumen content | | | | | | | | |
|---|---|---|---|---|---|---|---|---|
| RUN | Bacterial count (decimal logarithm) | | | | | | | |
| | total aerob | total thermo | total spores | Enterococci* | Coliforms* | Clostridia? | Salmonella; | NOTES |
| 1 b | 6.78 | 4.60 | 4.23 | 5.69 | 5.54 | <1 | - | Rumen |
| 2 b | 7.0 | 5.0 | 4.48 | 6.0 | 5.60 | <1 | - | content |
| 3 b | 6.48 | 5.0 | 5.08 | 5.81 | 5.60 | 2.48 | - | only |
| 4 b | 8.23 | 5.0 | 4.8 | 6.08 | 3.0 | 2.18 | - | poultry |
| 5 b | 8.65 | 5.48 | 5.48 | 6.43 | 3.60 | 2.18 | - | litter only |
| 6 b | 7.84 | 5.0 | 5.08 | 6.68 | 5.0 | 2.30 | - | mixture |
| b | 8.26 | 5.0 | 4.70 | 6.30 | 4.95 | 2.11 | - | |
| a | 3.70 | 3.70 | 2.48 | 2.40 | <1 | <1 | - | |
| a | 5.70 | 2.70 | 2.48 | 2.30 | <1 | <1 | - | |
| a | 4.50 | 2.23 | 2.58 | <1 | <1 | <1 | - | |
| a | 3.11 | 1.00 | 3.00 | <1 | <1 | <1 | - | |
| a | 4.90 | 2.30 | 3.08 | 2.95 | <1 | <1 | - | |
| a | 3.65 | 2.70 | 2.70 | 2.48 | <1 | <1 | - | |
| a | 5.36 | 1.30 | 2.60 | 2.0 | <1 | <1 | - | |
| a | 5.30 | 2.0 | 2.0 | <1 | <1 | <1 | - | |
| a | 3.34 | 1.03 | 2.34 | <1 | <1 | <1 | - | |
| a | 2.48 | 1.30 | 2.11 | <1 | <1 | <1 | - | |
| Note: in run 6, samples were collected from 10 different locations in the fermentation units. | | | | | | | | |

EXAMPLE IV: Artificial contamination with Salmonella Bacteria.

Poultry litter was contaminated in the laboratory with pure fresh (24 hour) culture of S. typhymurium at a rate of $10^6$ CFU/g., packed into bags resisting the fermentation process and inserted in a 30 ton fermentation unit at 6 different locations. At the end of the process the bags were recovered, opened and examined (using peptone and an inoculum of 100 g.) for the presence of Salmonella. No Salmonella could be detected in any of the treated material.

EXAMPLE V: Field trials.

The feed products of the present invention were utilized in 11 different farms in various parts of Israel, in order to evaluate possible damage to cattle fed with these products. The latter were utilized mainly for the feeding of female calves, and constituted 15-40% of the total diet. Only in one farm was some disturbance recorded, in the form of slight diarrhea. Detailed results are given in Table D; in addition to these results, no metabolic disorders were recorded in any of the animals fed with the product produced according to the process of the invention.

## TABLE D

| Field Trials | | | | | | | | |
|---|---|---|---|---|---|---|---|---|
| FARM | No. of heads in farm | | | | Duration of feeding (months) | Problems | | |
| | cows | heifers | f. calves | m. calves | | Vet./Med. | fertility | killed |
| 1 | 173 | 87 | 205 | | 24 | none | few | none |
| 2 | 270 | 70 | 160 | | 36 | none | none | none |
| 3 | 280 | 90 | 190 | | 36 | none | none | none |
| 4 | 280 | | 260 | | 36 | none | none | none |
| 5 | 270 | 75 | 160 | | 36 | ? | delays | none |
| 6 | 600 | | 400 | 200 | 36 | none | } | none |
| 7 | 230 | 100 | 290 | 180 | 14 | none | none | none |
| 8 | 275 | 90 | 245 | 160 | 18 | none | none | 3 |
| 9 | 200 | 60 | 180 | 180 | 6 | none | none | none |
| 10 | 270 | | 127 | 127 | 14 | none | none | none |
| 11 | 280 | | 240 | 140 | 6 | none | none | none |

?diarrhea

} in past

EXAMPLE VI: Computerized pulsing of air in fermentation process.

A mixture of 26,250 kg. poultry litter, 1,050 kg. food waste and 150 kg. lime was placed in each of a pair of containers, the supply of air to which was regulated by a computerized system as described above with reference to Fig. 6. In this Example, the computer was pre-set to terminate the process 48 hours after a temperature of 70°C was attained. Table E gives exemplary details of the temperature control of the feed by pulsing air for specified periods into the containers at stated intervals between pulses, the duration of the pulses and the intervals between them being determined by computer on the basis of the preceding increase (or decrease) in the sensed temperatures. The obtained product was substantially free of pathogenic microorganisms and was suitable for cattle feed.

TABLE E

| Temperature control in fermentation process. | | | | | | |
|---|---|---|---|---|---|---|
| DAY | TIME | TEMPERATURE | | | AIR PULSES | |
| | (hrs:mins:secs) | sensor #* | | | interval (mins.) | duration (secs.) |
| | | 1 | 2 | 3 | | |
| 1 | 12: 23: 39 | (ambient) | | | 3 | 60 |
| | 12: 26: 00 | 41.4 | 41.4 | 45 | 2 | 60 |
| | 14: 13: 14 | 45.4 | 44.8 | 49.7 | 2 | 60 |
| | 16: 39: 09 | 50.5 | 49.4 | 55.7 | 2.5 | 60 |
| | 20: 24: 12 | 54.3 | 55.3 | 60.5 | 2.5 | 60 |
| 2 | 00: 16: 28 | 57.2 | 60 | 64.6 | 2.5 | 60 |
| | 03: 16: 31 | 60.7 | 64.5 | 69.5 | 2.5 | 60 |
| | 07: 16: 32 | 63.8 | 66.1 | 69.3 | 2.5 | 60 |
| | 07: 31: 31 | 64.1 | 66.3 | 69.2 | 4.5 | 60 |
| | 13: 47: 21 | 69.2 | 70.4 | 76.4 | 4 | 60 |
| | 13: 59: 54 | 70.3 | 70.5 | 76.4 | 6 | 60 |
| | 14: 55: 41 | 70.1 | 71.4 | 77.4 | 8 | 60 |
| | 20: 55: 43 | 71.1 | 75.4 | 80.2 | 10 | 50 |
| | 23: 55: 44 | 73.7 | 78.5 | 80.9 | 10 | 40 |
| 3 | 00: 46: 06 | 74.2 | 79 | 80.9 | 10 | 24 |
| | 06: 46: 08 | 78 | 83 | 81 | 15 | 24 |
| | 13: 16: 54 | 80.3 | 85 | 80.7 | 20 | 24 |
| | 19: 31: 53 | 79 | 83.8 | 82.5 | 20 | 24 |
| 4 | 00: 31: 29 | 78.4 | 83.6 | 83.3 | 20 | 24 |
| | 06: 31: 33 | 77.9 | 83.6 | 84.2 | 20 | 24 |

* sensor #1 was movable and was placed in one of a number of optional locations as desired by the operator; sensor #2 was located in an upper section of the container; sensor #3 was located in a lower section of the container.

## ADVANTAGES OF THE INVENTION

A plant employing the present process can be built from units, such as fermentation chamber 10, (Figs. 2-5), of any size and number to suit local requirements; investment can be gradual in relation to supply and demand. As described above in conjunction with Fig. 7, the process can be readily adapted to computerized control, and a single computerized control unit can control a small or large number of processing units; also, it consumes local waste products which otherwise would tend to cause an environmental nuisance. Not least, the present process is economical when compared with possible alternatives; it can be sustained without the input of large amounts of energy from external sources, and it can be carried out in districts where large amounts of fuel are not readily available.

Another advantage is that it is economical in the sense that waste materials are utilized. Moreover, although no determinations of the effect on viruses has been carried out, it is known that viruses in oven-roasted turkey breasts are eliminated by heating for a very short time at 60°C; since in a particular embodiment of the present invention the starting materials can be raised slowly (e.g. over 48 hours) from ambient temperature to the recommended temperature range, it seems likely that the present process will result inter alia in the destruction of Newcastle Disease virus.

Also, in view of the eliminative effect of the present process on Clostridium welchii, it is probable that the effect on Cl. botulinum will be similar; furthermore, if in accordance with an embodiment of the invention a temperature of above 82°C is maintained for at least 48 hours, these conditions should be sufficient for denaturation of exotoxins of Cl. botulinum.

It will also be appreciated that the product is clean and unpolluted, and free of noxious odors. Moreover, it is believed that in the course of the process of the invention nonprotein nitrogen is transformed to high-grade microbial nitrogen, and that the high temperature of treatment improves the properties of starch and protein in the product so that they can be readily broken down in the digestive system. At a minimum, the nutritional value of the product will generally not be less than that of the raw materials and in many cases it will have a superior nutritional value.

It will be appreciated by persons skilled in the art that the present invention is not limited to what has been particularly shown and described, by way of example, hereinabove. The scope of the present invention is limited, rather, solely by the claims below.

## Claims

1. A process for the manufacture of feed for livestock and poultry which comprises: subjecting moist organic waste material to aerobic fermentation conditions at an operating temperature within the range of 70 to 85°C, until it is substantially free from pathogenic microorganisms.

2. Process according to claim 1, wherein said moist organic material comprises between 50 and 90 wt.% dry material.

3. Process according to claim 1, wherein said material comprises faeces.

4. Process according to claim 3, wherein said faeces constitutes at least 50% of said material on a dry weight basis.

5. Process according to claim 1, wherein lime is present in said material in an amount of between 0.1 and 1.0 wt.% on a dry weight basis.

6. Process according to claim 1, wherein said operating temperature is maintained by controlling access of air to said moist organic waste material.

7. Process according to claim 6, wherein additionally, said moist organic waste material is raised from ambient temperature to said operating temperature by controlling access of air thereto.

8. A process for the manufacture of feed for livestock and poultry which comprises: subjecting moist organic waste material to aerobic fermentation conditions for one to two days at an operating temperature within the range of 70 to 85°C.

9. Process according to claim 8, wherein said moist organic material comprises between 50 and 90 wt.% dry material.

10. Process according to claim 8, wherein said dry material comprises faeces.

11. Process according to claim 10, wherein said faeces constitutes at least 50% of said material.

12. Process according to claim 9, wherein lime is present in an amount between 0.1 and 1.0 wt.% of said material on a dry weight basis.

13. Process according to claim 8, wherein said operating temperature is maintained by controlling access of air to said moist organic waste material.

14. Process according to claim 13, wherein additionally, said moist organic waste material is raised from ambient temperature to said operating temperature by controlling access of air thereto.

15. Process according to either claim 2 or claim 9, wherein said material comprises 55 to 99.9wt.% poultry litter, 0 to 44.9wt.% other organic waste material and 0.1 to 1.0wt.% lime, on a dry weight basis.

16. Process according to claim 15, wherein said operating temperature is maintained by controlling access of air to said moist organic waste material.

17. Process according to claim 16, wherein additionally, said moist organic waste material is raised from ambient temperature to said operating temperature by controlling access of air thereto.

18. Process according to claim 15, wherein said aerobic conditions include supplying air in intermittent pulses to said moist organic waste material, the temperature of which is raised from ambient temperature to said operating temperature by control of said intermittent air supply, and said operating temperature is also maintained by control of said intermittent air supply.

19. Process according to claim 18, which includes the steps of monitoring the temperature at a plurality of points in said moist organic waste material at predetermined time intervals, and adjusting in a predetermined manner said intermittent air supply as to the intervals between pulses and as to the length of each pulse, in order to maintain differences in the temperature monitored at successive time intervals within predetermined limits.

20. Process according to claim 18, which includes the steps of monitoring both the oxygen content of the ambient gases, and the temperature, at a plurality of points in said moist organic waste material at predetermined time intervals, and adjusting in a predetermined manner said intermittent air supply as to the intervals betwen pulses and as to the length of each pulse, when the oxygen content of the preceding pulse has been depleted to a predetermined extent, in order to maintain differences in the temperature monitored at successive time intervals within predetermined limits.

21. Process according to claim 19, which employs programmed computerized control means, in such manner that said step of adjusting in a predetermined manner comprises control by output from said means, and the input to said means comprises data obtained from said monitoring step.

22. Process according to claim 20, which employs programmed computerized control means, in such manner that said step of adjusting in a predetermined manner comprises control by output from said means, and the input to said means comprises data obtained from said monitoring step.

23. Process according to claim 15, wherein said other waste organic material is selected from the group consisting of fermentable abattoir waste and kitchen waste, the process includes the preliminary steps of (i) subjecting the initial material(s) to thorough mixing, (ii) removal of injurious items therefrom, and (iii) if desired aging the resultant mixture for up to about three hours, and said aerobic conditions include supplying air in intermittent pulses to said moist organic waste material, the temperature of which is raised from ambient temperature to said operating temperature over a period of about 48 to 64 hours by control of said intermittent air supply, and said operating temperature is also maintained for a period of at least 32 hours by control of said intermittent air supply.

24. Process according to claim 23, which includes the steps of monitoring the temperature at a plurality of points in said moist organic waste material at predetermined time intervals, and adjusting in a predetermined manner said intermittent air supply as to the intervals between pulses and as to the length of each pulse, in order to maintain differences in the temperature monitored at successive time intervals within predetermined limits.

25. Process according to claim 23, which includes the steps of monitoring both the oxygen content of the ambient gases, and the temperature, at a plurality of points in said moist organic waste material at predetermined time intervals, and adjusting in a predetermined manner said intermittent air supply as to the intervals between pulses and as to the length of each pulse, when the oxygen content of the preceding pulse has been depleted to a predetermined extent, in order to maintain differences in the temperature monitored at successive time intervals within predetermined limits.

26. Process according to claim 24, which employs programmed computerized control means, in such manner that said step of adjusting in a predetermined manner comprises control by output from said means, and the input to said means comprises data obtained from said monitoring step.

27. Process according to claim 25, which employs programmed computerized control means, in such manner that said step of adjusting in a predetermined manner comprises control by output from said means, and the input to said means comprises data obtained from said monitoring step.

28. A system for the manufacture of feed for livestock and poultry comprising:
means for containing a volume of organic waste material; and
means associated with said means for containing for aerobically fermenting said volume of organic waste material.

29. A system according to claim 28, and wherein said means for aerobically fermenting comprises means for supplying air to said volume of waste material and thereby controlling the temperature of said waste material.

30. A system according to claim 29, and wherein said means for containing comprises at least one insulated container.

31. A system according to either of claims 29 or 30, and wherein said means for supplying air is operative to maintain said volume of waste material at a temperature within the range of 70 to 85°C for at least a predetermined period of time, until said volume of waste material is substantially free from pathogenic microorganisms.

32. A system according to claim 31, and also including means for monitoring the temperature of said waste material.

33. A system according to claim 32, and wherein said means for monitoring comprises at least one thermocouple device arranged at a predetermined location within each of said at least one insulated container for providing a signal output corresponding to the temperature of said waste material in each container.

34. A system according to claim 33, and also including signal receiving and processing means for receiving said signal output and determining a rate of temperature change within each of said at least one container, said receiving and processing means including means for evaluating said determined rate of temperature change and, in accordance with said evaluation, for selectively providing output signals to said means for supplying air so as to maintain the temperature of said waste material within said temperature range.

35. A system according to claim 34, and also including means for monitoring the oxygen level within each of said at least one container.

36. A system according to claim 35, and wherein said oxygen monitoring means includes means for providing a signal output to said signal receiving and processing means corresponding to an observed level of oxygen within each of said at least one container.

37. A system according to claim 36, and wherein said evaluation means is also operative to evaluate the observed level of oxygen within said at least one container and to selectively provide output signals to said means for supplying air in accordance with said evaluation.

38. A system according to any of claims 30-37, and wherein said container defines an inspection port facilitating sampling of said waste material during fermentation.

39. Process according to claim 1 and substantially as hereinbefore described.

IN

24

10

14

26

OUT

FIG.1

FIG.2

FIG.4

FIG.3

FIG.5

EP 0 378 507 A1

FIG.6

APPENDIX I

```
DECLARE FUNCTION NumberForControl% (What%, R%)
DECLARE SUB EveryQuarter (ProcessTour%, WriteTime%, WriteDrive!,
ThePrinter%, Quarter%)
DECLARE SUB SetHour (WriteTime%, WriteDrive!)
DECLARE SUB Up70Degree (seventyDegree%, hot!, LastQuarter!)
DECLARE SUB PicoIn (Drive$, Onoff$, OnTim%(), OffTim%())
DECLARE SUB ControlOutput (Onoff$, OnTim%(), OffTim%(), Parameter!())
DECLARE  SUB ProcessControl (c%,  Container()  AS  ANY,  Onoff$,
OnTim%(), OffTim%(), Parameter!())
DECLARE SUB ReadAllChanel (Channel!(), ForCom!, FlagRead%, Parameter!())


  TYPE THERMO
   Temperature AS SINGLE
   Quarter1    AS STRING * 5
   Quarter2    AS STRING * 5
   Quarter3    AS STRING * 5
   Quarter4    AS STRING * 5
   HeatQuart1  AS SINGLE
   HeatQuart2  AS SINGLE
   HeatQuart3  AS SINGLE
   HeatQuart4  AS SINGLE
   Lasthour1   AS SINGLE
   Lasthour2   AS SINGLE
   Lasthour3   AS SINGLE
   Lasthour4   AS SINGLE

  END TYPE

  TYPE Process
     ' The Configuration of Three Thermometer
   ThFirst     AS INTEGER
   ThSecond    AS INTEGER
   ThThree     AS INTEGER
     'the control -thermometer, the Control help thermometer and Verification
   ThControl   AS INTEGER
   HeatFirst   AS THERMO
   HeatSecond  AS THERMO
   HeatThree   AS THERMO
     'the on/off times
   Firstmin    AS INTEGER
   FirstSec    AS INTEGER
   Second      AS INTEGER
   Minute      AS INTEGER
   OnBefore    AS INTEGER
   OffBefore   AS INTEGER
     'the Heat before one or two Process Control
   Before1     AS SINGLE
   Before2     AS SINGLE
     ' the rate of increase
   Down        AS INTEGER
   UpNow       AS SINGLE
   UpQuarter   AS SINGLE
   UpHour      AS SINGLE
```

```
    Problem      AS INTEGER
    Numbprob     AS INTEGER
     'Process Data
    InDay        AS STRING * 10
    Number       AS INTEGER
    Up70         AS INTEGER
    Icaf         AS INTEGER
    Dry          AS SINGLE
    WriteDrive   AS SINGLE
    Cold         AS INTEGER
  END TYPE


CONST False = 0
CONST True = NOT False
CONST NumberDown = 60

DIM SHARED NumberContainer%
REDIM Container(1)    AS Process
DIM OnTim%(30), OffTim%(30)
DIM Parameter(10)
DIM Channel(90)
DIM WriteTime%
DIM Tim%
DIM Interval%
DIM ProcessTour%
DIM ThePrinter%
DIM Quarter%
DIM OneQuarter%
DIM TimeDown%
DIM SHARED Drive$

OPEN Drive$ + "Process.mat" FOR RANDOM AS 1 LEN = LEN(Container)
lg& = LOF(1)
NumberContainer% = lg& / LEN(Container)
CLOSE #1

REDIM SHARED Container(NumberContainer%)   AS Process

WriteTime% = 4 'Every 4 Hour Write on the Process File

CLS

PicoIn "\mamat\", Onoff$, OnTim%(), OffTim%()
ControlOutput Onoff$, OnTim%(), OffTim%(), Parameter()
ReadAllChanel Channel(), ForCom, FlagRead%, Parameter()


 'Every 30 Second
ON TIMER(30) GOSUB ROUTINE I
TIMER ON

Interval% = (15 * 60)

 DO
```

```
      OnScreen Tim%, Interval%, Channel!(), ScreenNumber%
    LOOP

  END

ROUTINE I

 ReadAllChanel Channel(), ForCom, FlagRead%, Parameter()

   'Set temp in Process Data
   FOR i% = 1 TO NumberContainer%

     IF  Channel!(Container(i%).ThFirst)                <
     Container(i%).HeatFirst.Temperature THEN
      Container(i%).Down = Container(i%).Down + 1
     ELSE
      Container(i%).Down = 0
     END IF

    Container(i%).HeatFirst.Temperature = Channel!(Container(i%).ThFirst)
    Container(i%).HeatSecond.Temperature = Channel!(Container(i%).ThSecond)
    Container(i%).HeatThree.Temperature = Channel!(Container(i%).ThThree)

     IF Container(i%).Down >= TimeDown% THEN
     ProcessControl i%, Container(), Onoff$, OnTim%(), OffTim%(), Parameter()
     END IF

   NEXT i%


 OneQuarter! = OneQuarter! + .5

   IF OneQuarter! = 15 THEN
    OneQuarter! = 0
    Time% = TIMER
    GO TO ROUTINE II
    EveryQuarter ProcessTour%, WriteTime%, WriteDrive!, ThePrinter%, Quarter%
   END IF

  RETURN

 END OF ROUTINE I

   SUB ControlOutput (Onoff$, OnTim%(), OffTim%(), Parameter()) STATIC

    PicoOutput = Parameter(2)
    IF PicoOutput = False THEN EXIT SUB
    FlagPicco% = False
    FlagTwo% = 0

    control$ = CHR$(194 + Parameter(2))
    CLOSE #2
    OPEN "COM2:4800,N,8,2,CS,DS,CD" FOR RANDOM AS #2
    PRINT #2, "000000000000000000000000000"
```

```
DO

  CLOSE #2
  OPEN "COM2:4800,N,8,2,CS,DS,CD" FOR RANDOM AS #2
   'Code for Controller
  PRINT #2, control$;

  t$ = TIME$


  FOR i% = 1 TO 3
    DO

      IF NOT EOF(2) THEN FlagPicco% = True

    LOOP UNTIL t$ <> TIME$ OR FlagPicco%
    IF FlagPicco% THEN EXIT FOR
  NEXT i%

  IF NOT FlagPicco% THEN
   LOCATE 24, 1
   COLOR 31, 0
   PRINT "Comunication Error .... ";
   COLOR 7, 0
   PRINT #2, "00000000000000000000000000"
  ELSE
   LOCATE 24, 1
   PRINT "                          ";
  END IF
 FlagTwo = FlagTwo% + 1

LOOP UNTIL FlagTwo% = 1 OR FlagPicco%

IF NOT FlagPicco% THEN EXIT SUB


DO
  errorA = 14630
  c$ = INPUT$(1, 2)
  errorA = 0
LOOP UNTIL EOF(2)

FOR i% = 1 TO 20
  x% = VAL(MID$(Onoff$, i%, 1))
  Y$ = CHR$(x%)

  IF Y$ = CHR$(2) THEN
    YPRE% = INT(OnTim%(i%) / 256)
    YSEC% = OnTim%(i%) MOD 256
    Y$ = Y$ + CHR$(YPRE%)
    Y$ = Y$ + CHR$(YSEC%)
    Y$ = Y$ + CHR$(INT(OffTim%(i%) / 256))
    Y$ = Y$ + CHR$(OffTim%(i%) MOD 256)
  END IF
```

```
      FOR R% = 1 TO LEN(Y$)
        PRINT #2, MID$(Y$, R%, 1);
        NEXT R%
     NEXT i%

      'Clear Rs232 Buffer
     CLOSE #2

   END SUB        ' PICOLOGER

ROUTINE II

   SUB   EveryQuarter (ProcessTour%,   WriteTime%,   WriteDrive!,
   ThePrinter%, Quarter%) STATIC

   ProcessTour% = ProcessTour% + 1
   IF ProcessTour% > 6 THEN ProcessTour% = 1
   BeforeTour% = ProcessTour%

   LOCATE 25, 20, 0
   PRINT "Just a Moment   , Process Number  "; ProcessTour%

    'Every hour
   Quarter% = Quarter% + 1
    IF Quarter% = 4 THEN
     Quarter% = 0

     SetHour WriteTime%, WriteDrive!

    END IF


    FOR i% = 1 TO NumberContainer%

       Container(i%).HeatFirst.Quarter4 = Container(i%).HeatFirst.Quarter3
       Container(i%).HeatFirst.Quarter3 = Container(i%).HeatFirst.Quarter2
       Container(i%).HeatFirst.Quarter2 = Container(i%).HeatFirst.Quarter1
       Container(i%).HeatFirst.Quarter1 = LEFT$(TIME$, 5)

       Container(i%).HeatFirst.HeatQuart4 = Container(i%).HeatFirst.HeatQuart3
       Container(i%).HeatFirst.HeatQuart3 = Container(i%).HeatFirst.HeatQuart2
       Container(i%).HeatFirst.HeatQuart2 = Container(i%).HeatFirst.HeatQuart1
       Container(i%).HeatFirst.HeatQuart1 = Container(i%).HeatFirst.Temperature

       Container(i%).HeatSecond.Quarter4 = Container(i%).HeatSecond.Quarter3
       Container(i%).HeatSecond.Quarter3 = Container(i%).HeatSecond.Quarter2
       Container(i%).HeatSecond.Quarter2 = Container(i%).HeatSecond.Quarter1
       Container(i%).HeatSecond.Quarter1 = LEFT$(TIME$, 5)

       Container(i%).HeatSecond.HeatQuart4 = Container(i%).HeatScnd.HeatQuart3
       Container(i%).HeatSecond.HeatQuart3 = Container(i%).HeatScnd.HeatQuart2
       Container(i%).HeatSecond.HeatQuart2 = Container(i%).HeatScnd.HeatQuart1
       Container(i%).HeatSecond.HeatQuart1 = Container(i%).HeatScnd.Temperature

       Container(i%).HeatThree.Quarter4 = Container(i%).HeatThree.Quarter3
```

```
      Container(i%).HeatThree.Quarter3 = Container(i%).HeatThree.Quarter2
      Container(i%).HeatThree.Quarter2 = Container(i%).HeatThree.Quarter1
      Container(i%).HeatThree.Quarter1 = LEFT$(TIME$, 5)

      Container(i%).HeatThree.HeatQuart4 = Container(i%).HeatThree.HeatQuart3
      Container(i%).HeatThree.HeatQuart3 = Container(i%).HeatThree.HeatQuart2
      Container(i%).HeatThree.HeatQuart2 = Container(i%).HeatThree.HeatQuart1
      Container(i%).HeatThree.HeatQuart1 = Container(i%).HeatThree.Temperature

      Container(i%).UpHour  = (Container(i%).HeatFirst.HeatQuart1)
      - (Container(i%).HeatFirst.Lasthour1)
      Container(i%).UpQuarter                                       =
      Container(i%).HeatFirst.Temperature
      Container(i%).HeatFirst.HeatQuart2

   NEXT i%

   'every 1/2 hour print graph on the printer
   ThePrinter% = ThePrinter% + 1
   IF ThePrinter% = 2 THEN
    ThePrinter% = 0
    Graphic
   END IF

   ' The Control Process for one Process of Each Set
   FOR i% = 1 TO SetNumber%
    ControlTour% = NumberForControl%(i%, ProcessTour%)
    ProcessControl ControlTour%,  Container(), Onoff$, OnTim%(),
    OffTim%(), Parameter()
   NEXT i%

END SUB
END ROUTINE II

FUNCTION NumberForControl% (What%, R%)

 NumberForControl% = (What% - 1) * 6 + R%

END FUNCTION

   PROCESS CONTROL:

SUB ProcessControl (c%,  Container() AS Process,  Onoff$,
    OnTim%(), OffTim%(), Parameter()) STATIC
 TheDry% = Container(c%).Dry
 TheControl% = Container(c%).ThControl

  SELECT CASE TheControl%
  CASE 1
   TheHeat! = Container(c%).HeatFirst.Temperature
  CASE 2
   TheHeat! = Container(c%).HeatSecond.Temperature
  CASE ELSE
  END SELECT
```

```
Second = Container(c%).Second
Minute = Container(c%).Minute

OnBef = Container(c%).OnBefore
OffBef = Container(c%).OffBefore

Before1 = Container(c%).Before1
Before2 = Container(c%).Before2

Up = TheHeat! - Before1
Up2 = Before1 - Before2

GoSec% = Container(c%).Firstmin
GoMin% = Container(c%).Firstmin
Numprob = Container(c%).Numbprob

Special$ = RIGHT$(STR$(Second), 1)

IF Special$ = "9" THEN EXIT SUB

  'New Process
  IF TheHeat! > 27 AND TheHeat < 49 THEN
    IF Container(c%).InDay <> DATE$ AND Minute > Container(c%).Firstmin THEN
      Container(c%).Minute = GoMin%
      Container(c%).Second = GoSec%
      GOTO EndControl
    END IF
  END IF

IF (TheHeat! < 25 OR Minute = 0) THEN EXIT SUB


  'The Dry Matter is < 60%
  IF TheDry% < 60 AND Numprob < 4 THEN
   Numprob = Numprob + 1
    IF Up > .6 THEN
      IF Second < 60 THEN
       Second = Second + 10
      ELSEIF Minute > 1 THEN Minute = Minute - .5
      END IF
    END IF
   GOTO EndControl
  END IF

  IF TheDry% < 60 AND Numprob = 4 THEN
   Numprob = 0
   TheControl% = 1
  END IF

  'The Dry Matter  is = > 60%

  'Problems
  Problem% = False
   IF Up < 0 AND Minute = GoMin% AND Second = 30 THEN
    Problem% = True
```

```
END IF
IF Up2 < .6 AND Up2 > 0 AND (TheHeat! < 54 OR TheHeat! > 66) THEN
 Problem% = True
END IF

IF Problem% THEN
 Container(c%).Problem = 1


  IF          Container(c%).HeatSecond.Temperature
 Container(c%).HeatFirst.Temperature > .6 THEN
   Container(c%).ThControl = 2
   Second = 60
   Minute = Minute - 2
   GOTO EndControl
  END IF

 Ver% = Container(c%).Numbprob
 Ver% = Ver% + 1
 IF Ver% > 2 THEN Ver% = 0

 Container(c%).Numbprob = Ver%

  IF Ver% = 1 THEN

    SELECT CASE Second
    CASE IS < 61
     Second = 68
     Minute = 2
    CASE 69 TO 74
     Second = 75
     Minute = 1
    CASE IS >= 75
     Second = 90
     Minute = 2
    CASE ELSE
    END SELECT

  END IF

  IF Ver% = 2 THEN
   Minute = 1
   IF Second = 90 THEN Second = 180
  END IF

ELSE
 Container(c%).Problem = 0
  ' end of Problem%
END IF

IF NOT Problem% THEN
  'Normal
  IF TheControl% = 2 AND TheHeat! > 68 THEN
   Second = 30
   Minute = 10
```

```
  GOTO EndControl
END IF

SELECT CASE TheHeat!

CASE IS < 47
  IF Container(c%).Cold THEN
   Container(c%).Cold = 0
   Second = 20
   Minute = 24
   GOTO EndControl
  ELSE
   EXIT SUB
  END IF

CASE 47 TO 67.9
  SELECT CASE Up
  CASE IS < 0
    IF Minute = GoMin% THEN
     Second = 30
    ELSEIF Minute = GoMin% + 2 THEN Minute = Minute - 2
    ELSE
     Minute = GoMin%
    END IF

  CASE IS < .3
    IF TheHeat! < 54 OR TheHeat! > 66 THEN
      IF Minute <> GoMin% THEN
       Minute = GoMin%
      ELSEIF Second < 75 THEN Second = Second + 15
      END IF
    ELSE
      IF Minute > GoMin% THEN
       Minute = Minute - 1
      ELSEIF Minute < GoMin% THEN Minute = Minute + 1
      ELSEIF Second > GoSec% THEN Second = Second   10
      ELSEIF Second < GoSec% THEN Second = Second + 10
      END IF
    END IF
  CASE IS > .5
    IF Up > Up2 THEN
      IF Second < 75 THEN
       Second = Second + 10
      ELSEIF Minute > 1 THEN Minute = Minute - .5
      END IF
    ELSE
     Second = OnBef
     Minute = OffBef
    END IF

  CASE 68 TO 69.9
    SELECT CASE Up
    CASE IS <= 0

    CASE IS < .3
```

```
       Second = 60
       Minute = 10
     CASE ELSE
       Second = 50
       Minute = 10
     END SELECT

CASE 70 TO 71.9
  Second = 40
  Minute = 10

CASE 72 TO 74.9
  Second = 30
  Minute = 12

CASE 75 TO 77.9
  Second = 24
  Minute = 14

CASE 78 TO 79.9
  Second = 24
  Minute = 14

CASE 78 TO 79.9
  Second = 24
  Minute = 14

CASE 78 TO 79.9
  Second = 24
  Minute = 14

CASE 78 TO 79.9
  Second = 24
  Minute = 14

CASE 78 TO 79.9
  Second = 24
  Minute = 16

CASE 80 TO 85
  Second = 24
  Minute = 20

CASE IS > 85
  IF Container(c%).Up70 > 48 THEN
    Container(c%).Cold = 1
    Second = 0
    Minute = 0
    MID$(Onoff$, c%, 1) = "1"
  END IF

CASE ELSE
END SELECT
END IF
```

```
EndControl:

    Container(c%).Second = Second
    Container(c%).Minute = Minute

    Container(c%).Before1 = TheHeat!
    Container(c%).Before2 = Before1

    Container(c%).Problem = Problem%
    Container(c%).Numbprob = Numprob

     IF Second <> OnTim%(c%) OR Minute <> OffTim%(c%) THEN
      OnTim%(c%) = Second
      OffTim%(c%) = Minute * 60
      Container(c%).OnBefore = Second
      Container(c%).OffBefore = Minute * 60

      ControlOutput Onoff$, OnTim%(), OffTim%(), Parameter()

     END IF

     PrintControl (c%)

    END SUB
     END PROCESS CONTROL


    READ TEMP SENSORS:
    SUB ReadAllChanel (Channel(), ForCom, FlagRead%, Parameter()) STATIC

     REDIM ReadCh(0 TO 9), NumerPico$(5)

     NumberOfPicco = Parameter(1)

     IF NumberOfPicco = 0 THEN EXIT SUB

     FlagRead% = 1

     NumerPico$(1) = CHR$(192)
     NumerPico$(2) = CHR$(193)
     NumerPico$(3) = CHR$(194)

     ForCom = True

     CLOSE #2: OPEN "COM2:4800,n,8,1,CS,DS,CD" FOR RANDOM AS #2

      FOR Y% = 1 TO NumberOfPicco

       IF Parameter(Y% + 2) = 0 THEN EXIT FOR

       PRINT #2, NumerPico$(Y%);

        FOR h% = 1 TO 2
           'go to routine to get data
```

```
     REDIM ReadCh(0 TO 9)
       ' GET DATA from 10 Channel..-

      FOR i% = 0 TO 9

        GOSUB ReadData
        IF COMER THEN EXIT FOR
        RES = 1000 * INT(char / 16) + 100 * (char AND 15)
        GOSUB ReadData
        RES = RES + 10 * INT(char / 16) + (char AND 15)
        GOSUB ReadData
        IF (char AND 128) <> 0 THEN RES = -RES
        KO = char AND 15
        IF KO > 7 THEN KO = KO - 16
        ReadCh(i%) = RES * 10 ^ KO
        ReadCh(i%) = INT(ReadCh(i%) * 10) / 10
         IF ABS(ReadCh(i%)) = 9999 THEN
          ReadCh(i%) = 0
         END IF
         IF ABS(ReadCh(i%)) > 4999 THEN
          COMER = True
          EXIT FOR
         END IF

       NEXT i%


       ' - no error
       IF COMER = False THEN EXIT FOR
       BEEP

      NEXT h%

      'set temp data in Channel data
      FOR A% = 0 TO 9
        IF ReadCh(A%) > 0 THEN
          Channel(A% + 10 * (Y% - 1)) = ReadCh(A%)
        END IF

      NEXT A%

     NEXT Y%

    FlagRead% = False
    ERASE ReadCh
    ERASE NumerPico$
    EXIT SUB


ReadData:
      COMER = False
      Comtime% = False
       'Delay
      DELCOM = 150
```

```
DO
  char = False
   IF LOC(2) > 0 THEN

      IF NOT EOF(2) THEN
       char$ = INPUT$(1, #2)

       char = ASC(char$)
      END IF
    ELSE
     Comtime% = Comtime% + 1
    END IF

  LOOP UNTIL Comtime% > DELCOM OR char
  IF char = False THEN
   COMER = True
  END IF

 RETURN


END SUB       'do ReadAllChanel

SUB SetHour (WriteTime%, WriteDrive!) STATIC

  IF TIMER - WriteDrive! >= WriteTime% THEN
    'Write on the File every  7 hours
   WriteOnDrive
   WriteDrive! = TIMER
  END IF

  'Add one hour if the Container is > 70 Degree
  FOR i% = 1 TO NumberContainer%
   LastQuarter! = VAL(Container(i%).HeatFirst.Quarter4)
   hot! = Container(i%).HeatFirst.Temperature

   Up70Degree Container(i%).Up70, hot!, LastQuarter!

  NEXT

  'Set the Temperature-Hour
  FOR i% = 1 TO NumberContainer%
   Container(i%).HeatFirst.Lasthour1 = Container(i%).HeatFirst.Lasthour2
   Container(i%).HeatFirst.Lasthour2 = Container(i%).HeatFirst.Lasthour3
   Container(i%).HeatFirst.Lasthour3 = Container(i%).HeatFirst.Lasthour4
   Container(i%).HeatFirst.Lasthour4 = Container(i%).HeatFirst.HeatQuart1
   Container(i%).HeatSecond.Lasthour1 = Container(i%).HeatSecond.Lasthour2
   Container(i%).HeatSecond.Lasthour2 = Container(i%).HeatSecond.Lasthour3
   Container(i%).HeatSecond.Lasthour3 = Container(i%).HeatSecond.Lasthour4
   Container(i%).HeatSecond.Lasthour4 = Container(i%).HeatSecond.HeatQuart1
   Container(i%).HeatThree.Lasthour1 = Container(i%).HeatThree.Lasthour2
   Container(i%).HeatThree.Lasthour2 = Container(i%).HeatThree.Lasthour3
   Container(i%).HeatThree.Lasthour3 = Container(i%).HeatThree.Lasthour4
   Container(i%).HeatThree.Lasthour4 = Container(i%).HeatThree.HeatQuart1
  NEXT
```

```
     ' End of Hour

END SUB

SUB Up70Degree (seventyDegree%, hot!, LastQuarter!)

 Time% = VAL(TIME$)

  IF hot! > 69.9 THEN
    IF Time% > LastQuarter% THEN
     seventyDegree% = seventyDegree% + Time% - LastQuarter%
    ELSE
     seventyDegree% = seventyDegree% + 1
    END IF
  END IF

END SUB
```

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | DD-A-223915 (INSTITUT FUR GEFLUGELWIRTSCHAFT) * the whole document * | 1-17, 28-39 | A23K1/10 A23N17/00 C12M1/36 A01C3/02 |
| Y | | 18-27 | |
| X | FR-A-2095623 (PORTLAND-ZEMENTWERKE HEIDELBERG) * page 2, lines 32 - 39 * <br><br> * page 4, lines 7 - 18 * <br> * page 5, line 26 - page 6, line 23 * | 1, 2, 6, 7, 28, 29, 31, 32, 39 | |
| Y | | 18-27 | |
| X | DE-A-2544960 (GOERGEN) * page 9, line 20 - page 10, line 24; claims 1-3 * <br> * page 6, lines 18 - 21 * <br> * page 5, paragraph 2 * | 1-17, 28-32, 39 | |
| X | US-A-3462275 (BELLAMY) * the whole document * | 1, 3, 8, 28, 39 | |
| A | * column 3, lines 51 - 57 * | 23 | TECHNICAL FIELDS SEARCHED (Int. Cl.5) |
| X | US-A-3857757 (HERRICK) * column 1, lines 1 - 13 * | 1, 3, 8, 28, 39 | A23N A23K A01C C12M |
| A | * column 2, line 23 - column 3, line 52; figure 1 * | 21, 22, 26, 27 | |
| X | WORLD PATENTS INDEX LATEST, Week 7934, August 1979, Nr. 79-62434B/34, Derwent Publications Ltd., London GB. & JP-54088872 -A- <br><br> * Abstract and figures * | 1, 2, 6-9, 13, 14, 28 <br><br> 29, 39 | |
| X | US-A-4302546 (SCHLICHTING JR.) * the whole document * | 1-4, 6-11, 13, 14, 28-32, 39 | |

The present search report has been drawn up for all claims

| Place of search | Date of completion of the search | Examiner |
|---|---|---|
| THE HAGUE | 23 APRIL 1990 | VAN OORSCHOT J.W.M. |

EPO FORM 1503 03.82 (P0401)

<table>
<tr><td colspan="2" style="text-align:center">European Patent Office</td><td style="text-align:center">EUROPEAN SEARCH REPORT</td><td>Application Number</td></tr>
</table>

EP    90 63 0017

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-3606449 (NEW BRUNSWICK SCIENTIFIC CO.,INC.)<br>* page 24, lines 5 - 11 *<br>* page 29, lines 11 - 14 * | 19-22,<br>24-27 | |
| A | DE-A-1918343 (NORDGARD)<br>* figure 1 * | 30, 38 | |
| A | WORLD PATENTS INDEX LATEST, Week 8801,<br>January 1988, Nr. 88-06138/01,<br>Derwent Publications Ltd., London GB.<br>& SU-A-1313498<br><br>* Abstract* | 33 | |
| A | EP-A-156176 (CETUS CORPORATION)<br>* page 1, line 1 - page 3, line 12 * | 19-27,<br>32-37 | |

TECHNICAL FIELDS
SEARCHED (Int. Cl.5)

The present search report has been drawn up for all claims

| Place of search | Date of completion of the search | Examiner |
|---|---|---|
| THE HAGUE | 23 APRIL 1990 | VAN OORSCHOT J.W.M. |

EPO FORM 1503 03.82 (P0401)

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
& : member of the same patent family, corresponding
    document